# EUROPEAN PATENT SPECIFICATION

(11) **EP 0 627 213 B1**
(45) Date of publication and mention of the grant of the patent: **04.03.1998**
(21) Application number: 93904308.9
(22) Date of filing: 12.02.1993
(51) Int. Cl.: A61K 7/06

(54) **HAIR GROWTH AND RESTORATION PROMOTER**
HAARWUCHSMITTEL
PROMOTEUR DE CROISSANCE ET DE RECONSTITUTION CAPILLAIRE

(30) Priority: 17.02.1992 JP 80549/92; 02.04.1992 JP 126615/92
(43) Date of publication of application: 07.12.1994
(73) Proprietor: NOMURA, Manabu, Miyazaki-gun, Miyazaki 889-16 (JP)
(72) Inventor: NOMURA, Manabu, Miyazaki-gun, Miyazaki 889-16 (JP)
(74) Representative: Stachow, E.-W., Prof. Dr.
(86) International application number: JP9300187
(87) International publication number: WO9315710

(56) References cited:
- JP-A- 2 279 618
- PATENT ABSTRACTS OF JAPAN vol. 13, no. 227 (C-600) & JP-A-01 038 012 (POLA CHEM IND INC)
- PATENT ABSTRACTS OF JAPAN vol. 17, no. 551 (C-1117) & JP-A-05 155 738 (KOSE CORP) & DATABASE WPI Derwent Publications Ltd., London, GB; AN 93-232240
- PATENT ABSTRACTS OF JAPAN vol. 6, no. 191 (C-127) & JP-A-57 102 809 (JIROU ISONO)

## Description

### TECHNICAL FIELD

This invention relates to a hair growth and restoration promoter having as an active ingredient, an extract drawn from at least one kind of orchidaceae plant selected from a group of Calanthe R. Br., Phaius Lour., and other genera having a pseudo bulb. More specifically, this invention relates to a hair growth and restoration promoter which is used for promotion of hair growth and restoration especially in a patient whose hair has fallen out, thinned out or been lost completely.

### BACKGROUND OF THE INVENTION

Conventionally, a variety of hair growth and restoration promoter have been used to cope with physiological symptoms such as balding and thinning of the hair, scalp disorders such as dandruff and seborrhea, and to prevent and treat these symptoms.

Generally, a preparation used for hair growth and restoration aims facilitating the circulation of the blood, activating the hair matrix cell, inhibiting the secretion of lipids from the scalp skin, and supplementing nutrition to the hair.

Prior art compositions which have attempted to achieve promotion of hair growth and restoration, have prepared compositions by combining glyceride pentadecanate, cepharanthin, Swertia extract liquid, soluble sustain, ginseng extract liquid, ℓ -menthol, isopropylmethylphenol, glycyrrhetinic acid, tocopherol acetate, hinokitiol, capsicum tincture, sweet grass extract, nictoinamide, salicylic acid, Takanaru, or etc.

The above-mentioned preparation are known for promoting hair growth and restoration. However, it is generally desired that a hair growth and restoration promoter not only promote hair growth, hair restoration, and prevent its falling out, but also be effective against the occurrence of dandruff or itch. It is also preferable that a hair growth and restoration promoter be safe without inflicting adverse reaction onto the scalp over a long period of time. A need for a safety and effective hair growth and restoration promoter has thus evolved.

By using conventional medications containing the aforementioned ingredients, severe loss of the hair is temporarily controlled, but thinning hair of hair is not, and the loss of hair recurs over time.

Therefore, conventional medications do not produce satisfactory results. Use of conventional medications and remedies in a patient whose hair had been lost entirely only produce downy hair about a half year after application, and no further hair growth can be expected.

Document Patent Abstracts of Japan, vol.23, no.227(C-600) discloses a promoter for hair restoration and hair growth.

In light of the foregoing, this inventor thought that the reason why the conventional hair growth and restoration promoter could produce unsatisfactory results, was because of shortage of ingredients which promote activation of the hair root and facilitate the rising (and strengthening) of the hair. Then he ascertained that an extract of a kind of plant has the effect to rise the hair and promotes its growth, as a result of keen studies to develop more effective hair growth and restoration promoter, and found that the vegetable extract drawn from the plant would become an effective hair growth and restoration promoter. Thus this invention has been completed.

Accordingly, it is an object of the present invention to provide a hair growth and restoration promoter to improve head hair, reduce the loss of hair, and to promote its growth and restoration for a short period of time. This object is achieved by applying the hair growth and restoration to a patient whose hair has badly fallen out, thinned out, or been lost wholly.

### DISCLOSURE OF THE INVENTION

The hair growth and restoration promoter of present invention comprises as an active ingredient, a vegetable extract drawn from at least one kind of orchidaceae plants selected from a group of Calanthe R. Br., and Phaius Lour., or other genera having a pseudo bulb, to attain the above-mentioned object.

### DETAILED DESCRIPTION OF THE PREFERRED EMBODIMENT

The hair growth and restoration promoter of present invention has as an active ingredient, a vegetable extract drawn from orchidaceae plants selected from a group of Calanthe R. Br., and Phaius Lour., or other genera having a pseudo bulb. Among these genera, extracts of Calanthe discolor Lindl., and Phaiusflavus (blume) Lindl. are found to have a more effective action. Further, a similar action is found in plants of the genera of orchidaceae plants having a pseudo bulb as a subterranean bulb. For example, orchidaceae plants having this pseudo bulb are classified into the following genera:

Calypso Salisb., Tipularia Nutt., Arundina Blume, Bletilla Reichb.fil., Liparis L. C. Richard, Eleorchis F. Maek., Malaxis Soland., Spathglottis Blume, Acanthophippium Yamamotoi Hayata, Tainia Blume, Hancockia Rolfe, Geodorum Jackson, Eulophia R. Br., Cremastra Lindl., Kitigorchis F. Maek., Oreorchis Lindl., Cymbidium Sw., Eria Lindl., Bulbophyllum Thou.

These orchidaceae plants can be used as an extract, even if these are produced from cultivated plants (for example, from greenhouse cultivation or transplant cultivation), or nature grown plants.

The active ingredient of the present invention is an extract drawn form the flower, root, stem or leaf of the above-mentioned orchidaceae plants, or its entire body, but an extract drawn from a subterranean stem of the plant is preferred since it exhibits superior properties. Especially, an extract drawn by using a hydrated alcohol as an extract solvent is preferred. Further, although the concentration of alcohol in the extract solvent is not defined, it is preferred that the solvent an aqueous solution containing 25 to 40% of alcohol by weight.

A manufacturing method of the vegetable extract is exemplified as follows:

Calanthe discolor Lindl. (from natural growth in a forest on a mountain in the suburbs of Miyazaki-city, Miyazaki-prefecture) is washed with water after removing mud and other matters adhering to its subterranean stem, and cut into pieces by cutter, etc. 300 gm of the pieces is immersed in 1,000 ml of an aqueous solution of 35% alcohol, and this extracting operation is continued by stirring for up to as long as 10 days. Then, the extract liquid is filtered to remove impurities and obtain a vegetable extract. This extract is an brownish solution having a mildly irritating odor.

Further, it is also possible to obtain a vegetable extract by the same operation using Phaius flavus (Blume) Lindl. (from natural growth in a forest on a mountain in the suburbs of Miyazaki-city, Miyazaki-prefecture) and Oreorchis pateus Lindl. (also from natural growth).

The hair growth and restoration promoter of the present invention is prepared by combining the obtained vegetable extract with benzoic acid as a preservative, vitamin E as an antioxidant, or vitamin C as a modifier of pH which is appropriately use in general cosmetics, or by diluting with water or alcohol, in order to increase its shelf life. The diluting ratio of the vegetable extract is not specially defined, but is recommended at a ratio of 5 to 40%, and preferably 10 to 30% in the total amount of the solution. In the concrete, 100 to 200 ml of vegetable extract is diluted to produce 1,000 ml of the total solution as a whole.

While the hair growth and restoration promoter of the present invention contains the aforementioned vegetable extract as an active ingredient, it is possible to combine as an additional ingredient, such as those used in conventional hair tonics. Ingredients which can be combined with the extract of the present invention include vitamins, amino acids, animal and plant oils, and sodium chloride.

Vitamins which can be used include Vitamin A, Vitamin B₁, Vitamin B_{2,} Niacin (nicotinic acid), Vitamin C, Vitamin E, sodium pantothenate, potassium pantothenate, biotin H (Vitamin H), etc.

One possible amino acid which can be used is Dopa, 2-amino-3-(3,4-dihydroxyphenyl) propanoic acid.

Some examples of animal and plant oils that can be used include horse oil, egg oil, olive oil, camellia oil, rape seed oil, sesame oil, and germ oil.

The above-mentioned ingredients that can be added in combination with the active ingredient (vegetable extract) of the present invention have the following general actions and effect:
Vitamin A is useful for the health of the mucous membrane and nerves. It is shown to have an excellent effect against follicular keratoderma;
Vitamin B's including vitamin B₁, vitamin B₂, and niacin which not only stabilize the nerves of skin and mucous membrane but also activate the head (scalp) skin, and is effective for cellular respiration;
Vitamin C promotes the metabolism of head skin;
Sodium pantothenate and potassium pantothenate improve the hair and prevent white hair;
Vitamin E acts to facilitate the circulation of the blood or reactivate the hair matrix cell; and
Biotin H (vitamin H) in a mixture of a plant oil facilitates the activation of the hair root and prevents the hair from falling out.

Dopa, 2-amino-3-(3,4-dihydroxyphenyl) propanoic acid, is oxidized to a precursor of melanin by enzymatic action and is then degraded to melanin for the synthesis of new skin and hair cells.

Animal and plant oils including horse oil, live oil, camellia oil, rape seed oil, sesame oil and germ oil act to soften the head (scalp) skin, and especially sesame oil in a mixture of biotin H activates the hair root and prevents the hair from falling out.

Sodium chloride through osmotic action facilitates the absorption of the vegetable extract from the head skin by adjusting the concentrating of its addition, and permeates each combined ingredient into the depth of the hair follicle to activate the hair root. In particular, the vegetable extract can promote the rising (and strengthening) of the hair, resulting in reducing the falling-out of the hair and growing it during a short period of time.

In the hair growth and restoration promoter of the present invention, combining appropriately one or more of these ingredients with said vegetable extract can produce a more effective hair growth and restoration promoter.

According to study by this inventor, biotin H, sesame oil and sodium chloride can be combined with the vegetable extract for a patient whose hair has badly fallen out or thinned out. Dopa can be further combined with the above-mentioned ingredients for a patient whose has been lost entirely. Germ oil can be further combined with the above-mentioned ingredients to shorten a rehabilitation period of the hair. In this case, the combining ratio of dopa, biotin H, sesame oil, germ oil or sodium chloride is not particular defined, but percentage of each ingredient to a gross weight ranges from 0.05 to 1.0% by weight of Dopa, preferably from 0.1 to 0.3% by weight; from 0.05 to 0.5% by weight of biotin H, preferably from 0.1 to 0.5% by weight; from 0.1 to 3.0% by weight of sesame oil, preferably from 0.5 to 1.5% by weight; from 0.1 to 3.0% by weight of germ oil, preferably from 0.5 to 1.5% by weight; and from 0.5 to 5.0% by weight of sodium chloride, preferably from 1.0 to 2.0% by weight. In the hair growth and restoration promoter comprising the vegetable extract according to the present invention, the combining ratio of these ingredients is not limited to the foregoing. The combining ratio may be changed depending on a target symptom to synergistically promote the action of hair growth.

According to further study by this inventor, it has been found that in order to effectively develop hair growth and restoration by use of the vegetable extract effectively and to attain the effect of hair growth and restoration for a short period of time, it is preferable to combine biotin H, sodium pantothenate, niacin, sodium chloride, and sesame oil and germ oil as plant oils, and horse oil as an animal oil with the vegetable extract.

The hair growth and restoration promoter of the present invention must contain the vegetable extract as an essential ingredient. However, it is needless to say that the aforementioned ingredients must not necessarily combine with other ingredients to achieve the object of the present invention.

In a preferred embodiment of the hair growth and restoration promoter of the present invention, as a result of study by this inventor in due consideration of the foregoing, the hair growth and restoration promoter contains at least biotin H, sesame oil and sodium chloride. In addition 100 to 200 ml of said vegetable extract is combined with 1,000 ml of a mixture of 0.1 to 0.15% biotin H by weight, 0.5 to 1.5% sesame oil by weight, and 1.0 to 2.0% sodium chloride by weight.

In the aforementioned embodiment of the present invention, when the hair growth and restoration promoter is applied to a patient whose hair has badly fallen out or thinned out, the osmotic action of sodium chloride permits sesame oil and biotin H to penetrate into the depth of hair follicle to activate the hair root. The vegetable extract as an active ingredient promotes the rising (and strengthening) of the hair and reduces its falling-out, thereby it is possible to grow the hair up for a short period of time.

In another embodiment of the present invention, the hair growth and restoration promoter contains at least Dopa, biotin H, sesame oil, and sodium chloride; and 100 to 200 ml of said vegetable extract is combined with 1,000 ml of a mixture of 0.1 to 0.3% Dopa by weight, 0.1 to 0.15% biotin H by weight, 0.5 to 1.5% sesame oil by weight, and 1.0 to 2.0% sodium chloride by weight.

In this embodiment, when the hair growth and restoration promoter is applied to a patient whose hair has been lost entirely, Dopa is degraded by enzymatic action in the body to produce melanin required for new skin and hair, and to rehabilitate head hair for a short period of time.

In yet another embodiment of the present invention, the hair growth and restoration promoter contains at least Dopa, biotin H, sesame oil, germ oil and sodium chloride; and 100 to 200 ml of said vegetable extract is combined with 1,000 ml of a mixture of 0.1 to 0.3% Dopa by wight, 0.1 to 0.15% biotin H by weight, 0.5 to 1.5% sesame oil by weight, 0.5 to 1.5% germ oil by weight,and 1.0 to 2.0 sodium chloride by weight.

In the above-mentioned embodiment of the present invention, the hair growth and restoration promoter displays superior results from the effect of the germ oil which softens the head skin thereby further shortening the rehabilitation period of the head hair.

### EXAMPLE

The invention is illustrated in more detail by reference to the following examples:

### Embodiment 1

3 gm of niacin, 3 gm of vitamin B₁, 3 gm of sodium pantothenate, 2 gm of potassium pantothenate, and 2 gm of benzoic acid are dissolved respectively in 50 ml of warm water. 14 gm of vitamin C are dissolved in 330 ml of warm water. 1 gm of vitamin A is dissolved in 40 ml of ethanol; 7 gm of sesame oil are dissolved in 60 ml of ethanol; and 5 gm of vitamin E are dissolved in 25 ml of ethanol. 10 ml of 92% glycerol are added to 10 gm of horse oil and dissolved in 30 ml of ethanol. 30 ml of 92% glycerol are added to 1 gm of biotin H and dissolved by heating in 70 ml of ethanol and 50 ml of water. Further, 20 gm of sodium chloride are dissolved in 105 ml of water. Then, these solutions are mixed, to which 0.01 gm of vitamin B₂ (Sigma) is added to obtain a mixed solution 1.

Then, 300 gm cut pieces of subterranean stem of Calanthe discolor Lindl. are immersed in 1,000 ml of 35% alcohol by weight and left to stand for more than 10 days to filter impurities and obtain an extract.

120 ml of said extract are added to mixed solution 1, and thus, a hair growth and restoration promoter is obtained.

### Embodiment 2

3 gm of niacin, 3 gm of vitamin B₁, 3 gm of sodium pantothenate, 2 gm of potassium pantothenate, and 2 gm of benzoic acid are dissolved respectively in 50 ml of warm water. 14 gm of vitamin C are dissolved in 80 ml of warm water. And 1 gm of Dopa, a kind of amino acid, is dissolved in 250 ml of warm water. 1 gm of vitamin A is dissolved in 40 ml of ethanol; 7 gm of sesame oil are dissolved in 60 ml of ethanol; and 5 gm of vitamin E are dissolved in 25 ml of ethanol. 10 ml of 92% glycerol are added to 10 gm of horse oil and dissolved in 30 ml of ethanol. 30 ml of 92% glycerol are added to 1 gm of biotin H and dissolved by heating in 70 ml of ethanol and 50 ml of water. 20 gm of sodium chloride are dissolved in 105 ml of water. Then, these solutions are mixed, to which 0.01 gm of vitamin B₂ (Sigma) is added to obtain a mixed solution 2.

Then, 300 gm cut pieces of subterranean stem of Calanthe discolor Lindl. are immersed in 1,000 ml of 35% alcohol by weight and left to stand for more than 10 days to filter impurities and obtain an extract.

120 ml of said extract are added to mixed solution 2, and thus, a hair growth and restoration promoter is obtained.

### Embodiment 3

3 gm of niacin, 3 gm of vitamin B₁, 3 gm of sodium pantothenate, 2 gm of potassium pantothenate, and 2 gm of benzoic acid are dissolved respectively in 50 ml of warm water. 14 gm of vitamin C are dissolved in 80 ml of warm water. And 1 gm of Dopa, is dissolved in 250 ml of warm water. 1 gm of vitamin A is dissolved in 40 ml of ethanol; 7 gm of sesame oil and 7 mg of germ oil are dissolved respectively in 30 ml of ethanol; and 5 gm of vitamin E are dissolved in 25 ml of ethanol. 10 ml of 92% glycerol are added to 10 gm of horse oil and dissolved in 30 ml of ethanol. 30 ml of 92% glycerol are added to 1 gm of biotin H and dissolved by heating in 70 ml of ethanol and 50 ml of water. 20 gm of sodium chloride are dissolved in 105 ml of water. Then, these solutions are mixed, to which 0.01 gm of vitamin B₂ (Sigma) is added to obtain a mixed solution 3.

Then, 300 gm cut pieces of subterranean stem of Calanthe discolor Lindl. are immersed in 1,000 ml of 35% alcohol by weight and left to stand for more than 10 days to filter impurities and obtain an extract.

120 ml of said extract are added to mixed solution 3, and thus, a hair growth and restoration promoter is obtained.

In the foregoing embodiments, the ratio of each ingredient to a gross weight is 0.1% by weight of Dopa, 0.1% by weight of biotin H, 0.7% by weight of sesame oil, 0.7% by weight of germ oil, or 2.0% weight of sodium chloride in the preparation of mixed solution 1, 2 or 3, but the mixed solution is not necessarily limited to this ratio, and it has been supported from experiments that if the ratio is 0.1 to 0.3% by weight of Dopa, 0.1 to 0.15 % by weight of biotin H, 0.5 to 1.5% by weight of sesame oil, 0.5 to 1.5% by weight of germ oil, or 1.0 to 2.0% by sodium chloride, the anticipated effect can be sufficiently attained.

Further, in the aforementioned embodiments, the amount of the vegetable extract that can be combined with 1,000 ml of mixed solution 1, 2, or 3 is 120 ml, but the vegetable extract is not necessarily limited to this amount. It has been supported from experiments that if the amount of the vegetable extract to be combined with 1,000 ml of mixed solutions 1, 2 or 3 is from 100 to 200 ml, the anticipated effect can still be achieved.

Furthermore, in each of said embodiments, use of the extract of subterranean stem of Calanthe discolor Lindl. can promote the rising (and strengthening) of the hair by its action as described hereinafter, but an extract drawn from a subterranean stem of Phaius (Blume) Lindl. or a subterranean stem of Oreorchis patens Lindl. is also found to have similar action. Therefore It is possible to use the subterranean stem of Phaius flavus (Blume) Lindl. or the subterranean stem of Oreorchis patens Lindl. in place of Calanthe discolor Lindl.

In this case, an extract from the subterranean stem of Phaius flavus (Blume) Lindl. or the subterranean stem of Oreorchis patens Lindl . can be drawn in the same operation as for the subterranean stem of Calanthe discolor Lindl.

### Embodiment 4

5 gm of niacin, 5 gm of sodium pantothenate, 17 gm of vitamin C, 1 gm of biotin H, 15 ml of sesame oil, 15 ml of germ oil, 20 ml of horse oil, and 24 gm of sodium chloride are used, and 150 ml of the extract drawn from the subterranean stem of Calanthe discolor Lindl. obtained in Embodiment 1 is mixed with and dissolved in 700 ml of water and 500 ml of alcohol to obtain a hair growth and restoration promoter. The method for mixing and dissolving each ingredient is subjected to that described in Embodiment 1.

Results from the use of a hair growth and restoration promoter according to the present invention are as follows:

The hair growth and restoration promoter obtained in Embodiment 3 is applied everyday to a patient whose hair had badly fallen out or thinned out.

As a result, a reduction in the loss of the hair is found 4 to 5 days after application, and hair growth is rejuvenated to cover his scalp skin over within 6 months after application.

Further, the hair growth and restoration promoter obtained in Embodiment 3 is applied everyday to a patient whose hair had been lost entirely. As result, downy hairs began growing over the bald spots in 2 to 3 months after application, and the hair continued to grow to cover the scalp over for a period of one year after application.

As mentioned above, the hair growth and restoration promoter of the present invention has an extract drawn from at least one kind of orchidaceae plants, selected from a group of Calanthe R. Br., or Phaius Lour., or other genera having a pseudo bulb. The foregoing composition of ingredients displays the excellent effects of activating the hair root, and promoting the rising (and strengthening) of the head hair through the action of the vegetable extract as an active ingredient, reducing the falling-out of the hair and promoting its growth for a short period of time.

### INDUSTRIAL APPLICABILITY

The hair growth and restoration promoter of this invention can be used for reducing the falling-out of the hair and promoting its growth and restoration in a patient whose hair has badly fallen out or thinned out, or been lost entirely.

## Claims

1. A hair growth and restoration promoter including as an active ingredient an extract drawn from ochidaceae plant characterized in that the orchidaceae plant is at least one selected from the group consisting of Calanthe R.Br. and Phaius Lour.

2. A hair growth and restoration promoter according to the claim 1 is characterized in that said orchidaceae plant of Calanthe R.Br. is Calanthe discolor Lindl.

3. A hair growth and restoration promoter according to the claim 1 is characterized in that said orchidaceae plant of Phaius Lour. is Phaius flavus (Blume) Lindl.

4. A hair growth and restoration promoter according to the claim 1 is characterized in that said extract is drawn from subterranean stem.

5. A hair growth and restoration promoter according to the claim 1 is characterized in that further ingredients are at least one of vitamins, at least one of amino acids, at least one of animal oils or plant oils, or sodium chloride.

## Patentansprüche

1. Haarwuchsmittel das als einen aktiven Bestandteil ein von Orchideenpflanzen gewonnenes Extrakt enthält, **dadurch gekennzeichnet**, daß die Orchideenpflanze wenigstens aus der Gruppe der Calanthe R.Br. und Phaius Lour ausgewählt ist.

2. Haarwuchsmittel nach Anspruch 1, **dadurch gekennzeichnet**, daß die Orchideenpflanze aus Calanthe R.Br. eine Calanthe discolor Lindl ist.

3. Haarwuchsmittel nach Anspruch 1, **dadurch gekennzeichnet**, daß die Orchideenpflanze aus Phaius Lour. eine Phaius flavus (Blume) Lindl ist.

4. Haarwuchsmittel nach Anspruch 1, **dadurch gekennzeichnet**, daß das Extrakt aus dem unterirdischen Stiel gewonnen ist.

5. Haarwuchsmittel nach Anspruch 1, **dadurch gekennzeichnet**, daß als weiterer Bestandteil wenigstens ein Vitamin, eine Aminosäure, ein tierisches oder pflanzliches Öl oder Natriumchlorid sind.

## Revendications

1. Promoteur de repousse et de croissance capillaire comprenant en tant que principe actif un extrait tiré de plantes orchidacées, caractérisé en ce que la plante orchidacée est au moins au nombre de une, choisie dans le groupe constitué par Calanthe R.Br. et Phaius Lour.

2. Promoteur de repousse et de croissance capillaire selon la revendication 1, caractérisé en ce que ladite plante orchidacée Calanthe R.Br. est Calanthe discolor Lindl.

3. Promoteur de repousse et de croissance capillaire selon la revendication 1, caractérisé en ce que ladite plante orchidacée Phaius Lour. est Phaius flavus (Blume) Lindl.

4. Promoteur de repousse et de croissance capillaire selon la revendication 1, caractérisé en ce que ledit extrait est tiré de tiges souterraines.

5. Promoteur de repousse et de croissance capillaire selon la revendication 1, caractérisé en ce que d'autres ingrédients sont, au moins une vitamine, au moins un amino-acide, au moins une huile animale ou végétale, ou du chlorure de sodium.
